# EUROPEAN PATENT APPLICATION

(11) **EP 4 464 294 A1**
(43) Date of publication of application: **20.11.2024**
(21) Application number: 23796038.0
(22) Date of filing: 06.04.2023
(51) Int. Cl.: A61F 13/494, A61F 13/49, A61F 13/496

(54) **PANTS-STYLE DISPOSABLE WEARABLE ARTICLE**

(30) Priority: 27.04.2022 JP 2022073756
(71) Applicant: Daio Paper Corporation, Ehime 799-0492 (JP)
(72) Inventor: NAKAMARU, Hikari, Shikokuchuo-shi, Ehime 799-0113 (JP); FUJIWARA, Yuto, Shikokuchuo-shi, Ehime 799-0113 (JP)
(74) Representative: Williams Powell
(86) International application number: PCT/JP2023/014235
(87) International publication number: WO 2023/210292

(57) **Abstract**

The problem is to suppress an influence exerted on a side portion of an absorber by a leg movement under the presence of a single type planar gather part. The above problem is solved by the following solution. A side flap 60 has a width being 0.1 to 0.5 times the maximum width of the absorber 56. The side flap 60 has a first gather elastic member 631 attached on a lateral side of side edges of the portions 54 with the maximum width of the absorber 56, placing a first distance d1 therebetween, a second gather elastic member 632 attached on a lateral side of the first gather elastic member 631, placing a second distance d2 therebetween, and third gather elastic members 633 attached repeatedly on a lateral side of the second gather elastic member 632, placing a third distance d3 therebetween. The second distance d2 is 1.5 to 5 times the third distance d3 and the first distance d1 is 0.3 to 0.8 times the second distance d2. The fineness of the first gather elastic member 631 is 0.95 to 1.05 times the fineness of the second gather elastic member 632 and is 0.95 to 1.05 times the fineness of the third gather elastic member 633 and at the same time, the stretch rate of the first gather elastic member 631 is 1.2 to 1.5 times the stretch rate of the second gather elastic member 632 and is 1.2 to 1.5 times the stretch rate of the third gather elastic member 633.

## Description

### Technical Field

The present invention relates to an underpants-type disposable wearing article provided with planar gather parts on both sides in a width direction of an inner member.

### Background Art

It is general that an underpants-type disposable wearing article such as an underpants-type disposable diaper, underpants-type sanitary article, and the like is provided with an outer member forming at least a lower torso portion of a front body part and a lower torso portion of a back body part, and an inner member including an absorber and being attached to the outer member so as to extend from the front body part to the back body part, and in the underpants-type disposable wearing article, both side edge portions of the outer member in the front body part and both side edge portions of the outer member in the back body part are bonded to each other, respectively so as to form side seal portions, thereby forming a waist opening and a pair of right and left leg openings.

In the outer member, elastic members are provided in a lower torso region determined as a range in a front-back direction including the side seal portions (a range extending in the front-back direction from the waist opening to upper ends of the leg openings), thus, elasticity along a width direction is imparted to the lower torso region. Due to such elasticity, the lower torso region fits well to a wearer's body surface.

In addition, in a general underpants-type disposable wearing article, for ensuring fitting thereof to peripheries of wearer's legs and for preventing so-called side leakage, side gather parts are provided at both side portions in a width direction of an inner member so as to fit to wearer's inner thighs (see, for example, Patent Literature 1 to 3).

Conventionally, for the side gather parts, various types of structures have been proposed. For example, gather parts rising from side portions of an inner member toward a top side, are generally referred to as three-dimensional gather parts and have an excellent leakage-prevention effect. On the other hand, gather parts, which are formed of side flaps extending laterally from an absorber (without being folded back toward a top side), are generally referred to as planar gather parts and mostly combined with three-dimensional gather parts for use.

In addition, a proposed specifically low-cost product is free of three-dimensional gather parts, although it is provided with planar gather parts (see, for example, Patent Literature 1 to 3). Such a single type planar gather part has, in a crotch part, a first portion and a second portion. Then, in a worn state, the first portion extends obliquely in an upward direction from a side edge of an absorber toward a root of a wearer's inner thigh and the second portion extends obliquely along the wearer's inner thigh in a downward direction from a tip (distal end) of the first portion. Further, the second portion has an elongated gather elastic member attached thereto along a front-back direction such that in a natural length state, the second portion contracts in the front-back direction due to contraction of the gather elastic member, and the second portion is also stretchable in the front-back direction due to stretching of the gather elastic member. Then, in a worn state, the second portion of the planar gather part is pressed against the wearer's inner thigh by a contraction force of the gather elastic member.

However, in the conventional single type planar gather part, there is a problem as follows. That is, when the second portion moves according to a leg movement during walking or the like, such a movement of the second portion tends to be transmitted to the absorber through the first portion because of no support by a three-dimensional gather part. Further, when the movement of the second portion is transmitted to the absorber through the first portion, loss of shape in a side portion of the absorber may be caused. Moreover, this loss of shape in the side portion of the absorber is likely to weaken a support by the planar gather part such that fitting of the second portion may be deteriorated. Therefore, it is desirable to improve the conventional single type planar gather part.

### Citation List

### Patent Literature

Patent Literature 1: JP 5303689 B
Patent Literature 2: JP 5400982 B
Patent Literature 3: JP 6986097 B
Patent Literature 4: JP 2021-000236 A

### Summary of Invention

### Technical Problem

Therefore, a main object of the present invention is, for example, to suppress an influence exerted on a side portion of an absorber by a leg movement under the presence of a single type planar gather part.

### Solution to Problem

An underpants-type disposable wearing article solving the above mentioned problem is as follows.

### <First aspect>

An underpants-type disposable wearing article free of three-dimensional gather parts rising toward a top side, including:
an outer member, which is provided integrally from a front body part to a back body part, or which is provided separately on the front body part and the back body part,
an inner member attached to a middle part in a width direction of the outer member and provided to extend from a position on a front side of a crotch part to a position on a back side of the crotch part,
a pair of side seal portions in which both side portions of the outer member in the front body part and both side portions of the outer member in the back body part are bonded to each other, respectively,
a waist opening formed by a front edge of the front body part and a back edge of the back body part, and
leg openings provided on both lateral sides of the inner member,
wherein the inner member includes an absorber provided to be continuous from a position on the front side of the crotch part to a position on the back side of the crotch part,
the absorber includes a portion with a maximum width on the front side of the crotch part and a portion with the maximum width on the back side of the crotch part,
a pair of side flaps are provided on both side portions of the inner member to project laterally on both sides in the width direction from both side edges of the absorber and extend in a front-back direction from a front end portion of the inner member to a back end portion thereof,
each of the side flaps includes a planar gather part provided to be continuous from a lateral side of the portion with the maximum width of the absorber on the front side of the crotch part to a lateral side of the portion with the maximum width of the absorber on the back side of the crotch part,
the planar gather part includes elongated gather elastic members incorporated therein along the front-back direction such that the planar gather part is contracted in the front-back direction due to contraction of the gather elastic members and stretchable in the front-back direction due to stretching of the gather elastic members,
the side flap has a width being 0.1 to 0.5 times the maximum width of the absorber;
the gather elastic members are composed of a first gather elastic member attached on a lateral side of side edges of the portions with the maximum width of the absorber such that there is a first distance between the first gather elastic member and the side edges of the portions with the maximum width, a second gather elastic member attached on a lateral side of the first gather elastic member such that there is a second distance between the second gather elastic member and the first gather elastic member, and third gather elastic members attached repeatedly to a side edge portion of the side flap on a lateral side of the second gather elastic member such that there is a third distance for each space between two adjacent members of the second gather elastic member and the third gather elastic members;
the first gather elastic member has a first fineness and is attached to the side flap at a first stretch rate;
the second gather elastic member has a second fineness and is attached to the side flap at a second stretch rate;
each of the third gather elastic members has a third fineness and is attached to the side flap at a third stretch rate;
the second distance is 1.5 to 5 times the third distance;
the first distance is 0.3 to 0.8 times the second distance; and
the first stretch rate is 0.95 to 1.05 times the second stretch rate and is 0.95 to 1.05 times the third stretch rate and at the same time, the first fineness is 1.2 to 1.5 times the second fineness and is 1.2 to 1.5 times the third fineness, the first fineness is 0.95 to 1.05 times the second fineness and is 0.95 to 1.05 times the third fineness and at the same time, the first stretch rate is 1.2 to 1.5 times the second stretch rate and is 1.2 to 1.5 times the third stretch rate, or the first fineness is 1.2 to 1.5 times the second fineness and is 1.2 to 1.5 times the third fineness and at the same time, the first stretch rate is 1.2 to 1.5 times the second stretch rate and is 1.2 to 1.5 times the third stretch rate.

### (Effect)

In the underpants-type disposable wearing article, each side flap including the planar gather part is secured to have a sufficiently large width, and additionally, the first gather elastic member is provided near the absorber, the second gather elastic member is provided at a position which is laterally separated from the first gather elastic member to some degree, and the third gather elastic members are provided repeatedly to the side edge portion of the side flap at short intervals. In addition, a force required for stretching the first gather elastic member provided near the absorber is stronger than a force required for stretching the second gather elastic member and is stronger than a force required for stretching the third gather elastic member. As a result, in an appropriate worn state, the following situation is caused. Precisely, in the crotch part, a portion, which is located between a side edge of the absorber and the second gather elastic member, extends obliquely in an upward direction toward a root of a wearer's inner thigh as a first portion, and a portion, which is located between the second gather elastic member and the side edge of the side flap, extends obliquely along the wearer's inner thigh in a downward direction as a second portion. Then, the first portion is raised due to contraction of the first gather elastic member by a stronger force than a force by which the second portion is raised, such that the first portion is unlikely to move. Thus, the second portion, which is located at the position separated from the first gather elastic member to some degree, is brought closely into contact with the wearer's inner thigh, while the second portion is supported by the first gather elastic member. Therefore, even if the second portion moves according to a leg movement, since a section, which is located between the first gather elastic member and the second gather elastic member, is deformed so as to have a buffer effect, it is unlikely to happen that the movement of the second portion is transmitted to the absorber through the first portion. Thus, a possibility of loss of shape in the side portion of the absorber can be reduced.

### <Second aspect>

The underpants-type disposable wearing article according to the first aspect,
wherein the absorber has a portion with a minimum width in the crotch part, and widening portions, one of which is provided between the portion with the minimum width and the portion with the maximum width on the front side so as to widen forward gradually, and the other of which is provided between the portion with the minimum width and the portion with the maximum width on the back side so as to widen backward gradually; and
a distance in the width direction between a side edge of the portion with the minimum width of the absorber and the first gather elastic member is 1.1 to 1.5 times the second distance.

### (Effect)

If the distance in the width direction between the side edge of the portion with the minimum width of the absorber and the first gather elastic member is too large, a portion, which is located between the side edge of the portion with the minimum width of the absorber and the first gather elastic member, tends to be concaved deeply to an underside such that excrement may remain on the side flap, a finger of the wearer's leg may be caught on the portion when the wearer puts on the wearing article, or the like. Therefore, configuration of the second aspect is preferable.

### <Third aspect>

The underpants-type disposable wearing article according to the first or second aspect,
wherein, a section, which is located between the first gather elastic member and the second gather elastic member, has a three layered structure formed of a top surface-nonwoven fabric layer, an underside surface-nonwoven fabric layer, and a liquid impervious film disposed therebetween;
the top surface-nonwoven fabric layer and the liquid impervious film are bonded to each other through a first hot melt adhesive;
the underside surface-nonwoven fabric layer and the liquid impervious film are bonded to each other through a second hot melt adhesive;
each of the top surface-nonwoven fabric layer and the underside surface-nonwoven fabric layer has a bending resistance of 0.2 to 2.2 mN.cm in the front-back direction and 0.05 to 0.5 mN.cm in the width direction according to 41.5° Cantilever Method defined by JIS L 1913: 2010;
the liquid impervious film has a bending resistance of 0.006 to 0.05 mN.cm in the front-back direction LD and 0.006 to 0.05 mN.cm in the width direction WD according to 41.5° Cantilever Method defined by JIS L 1913: 2010;
an application amount of the first hot melt adhesive is 1 to 10 g/m²; and
an application amount of the second hot melt adhesive is 1 to 10 g/m².

### (Effect)

Since the section, which is located between the first gather elastic member and the second elastic member, has the buffer effect, it is preferable that this section has low rigidity as in the third aspect.

### <Fourth aspect>

The underpants-type disposable wearing article according to any one of the first to third aspects,
wherein the absorber has a portion with a minimum width in the crotch part, and widening portions, one of which is provided between the portion with the minimum width and the portion with the maximum width on the front side so as to widen forward gradually, and the other of which is provided between the portion with the minimum width and the portion with the maximum width on the back side so as to widen backward gradually; and
when the underpants-type disposable wearing article is placed on a horizontal plane while the back body part is located on a bottom side and only the outer member is stretched from a natural length state to a spread state along the horizontal plane for viewing the underpants-type disposable wearing article from above,
   in an outer shape-line of the crotch part, an acute intersecting angle α between the front-back direction and an imaginary line connecting a position of a side edge of the absorber and a position of the first gather elastic member is 15 to 40 degrees, an acute intersecting angle γ between the front-back direction and an imaginary line connecting two positions of the third gather elastic members adjacent to each other is 50 to 90 degrees, and an acute intersecting angle β between the front-back direction and an imaginary line connecting the position of the first gather elastic member and a position of the second gather elastic member keeps a relationship: α<β<γ.

### (Effect)

Configuration of the fourth aspect is preferable, because the buffer effect explained before becomes more excellent.

### Advantageous Effects of Invention

As stated above, the present invention provides advantages, for example, it is possible to suppress an influence exerted on a side portion of an absorber by a leg movement under the presence of a single-type planar gather part.

### Brief Description of Drawings

Fig. 1 is a plan view illustrating a top surface of an underpants-type disposable diaper in a spread state.
Fig. 2 is a plan view illustrating an underside surface of the underpants-type disposable diaper in the spread state.
Fig. 3 is a cross-sectional view taken along 3-3 line of Fig. 1.
Fig. 4 is a cross-sectional view taken along 4-4 line of Fig. 1.
Fig. 5 is a cross-sectional view taken along 5-5 line of Fig. 1.
Fig. 6 is a plan view illustrating a top surface of an inner member.
Fig. 7 is a perspective view of an underpants-type disposable diaper viewed from an obliquely front lower side.
Fig. 8 is a cross-sectional view illustrating a worn state of a main part of the underpants-type disposable diaper.
Fig. 9 is a front view schematically illustrating inclination of respective portions of a side flap.

### Description of Embodiments

Hereinafter, as an example of the above explained underpants-type disposable wearing article, an underpants-type disposable diaper will be described with reference to the accompanying drawings. Note that respective constituent members adjacent in a thickness direction are fixed or bonded to each other as necessary in a similar manner to a known diaper also at a portion other than a fixed or bonded portion described below. Each of dotted pattern regions in the cross-sectional views indicates an adhesive such as a hot melt adhesive as a fixing or bonding means. The hot melt adhesive can be applied by a known method such as slot application, bead application into a continuous line or dot line, spray application into a spiral shape, Z shape, (regular or irregular) wave shape, etc., pattern coating (transfer of a hot melt adhesive by a letterpress method) or the like. Alternatively, a fixed portion for an elastic member is formed, instead of or in addition to the above applications of the hot melt adhesive, by application of the hot melt adhesive to an outer peripheral surface of the elastic member, and the elastic member can be fixed to a member located adjacent thereto. Examples of the hot melt adhesive include an EVA-based agent, pressure sensitive adhesive rubber-based agent (elastomer-based agent), polyolefin-based agent, and polyester/polyamide-based agent, and these can be used without particular limitation. As a fixing or bonding means for bonding respective constituent members to each other, a means by material welding such as heat sealing or ultrasonic sealing also can be used. In a portion where a pervious property in a thickness direction is required, the respective constituent members adjacent in the thickness direction are fixed or bonded to each other in an intermittent pattern. For example, when such intermittent fixing or bonding is performed with a hot melt adhesive, intermittent pattern application in a spiral shape, Z shape, wave shape, or the like can be suitably used, and when application is performed in a range larger than an application width by one nozzle, intermittent pattern application in a spiral shape, Z shape, wave shape, or the like can be performed with or without a space in a width direction. As a bonding means for bonding the respective constituent members to each other, a means by material welding such as heat sealing or ultrasonic sealing also can be used.

In addition, as a nonwoven fabric in the following description, a known nonwoven fabric can be appropriately used according to a site or a purpose. Examples of a constituent fiber of the nonwoven fabric include a synthetic fiber such as a polyolefin-based fiber including polyethylene and polypropylene, polyester-based fiber, or polyamide-based fiber (including a composite fiber such as core-sheath in addition to a single component fiber), regenerated fiber such as rayon or cupra, and natural fiber such as cotton, and any of these fibers can be selected without particular limitation. It is also possible that these fibers are mixed and used. In order to enhance flexibility of the nonwoven fabric, it is preferable to use a crimped fiber as the constituent fiber. In addition, the constituent fiber of the nonwoven fabric may be a hydrophilic fiber (including a fiber that has become hydrophilic by a hydrophilizing agent), hydrophobic fiber, or water-repellent fiber (including a fiber that has become water-repellent by a water repellent agent). In addition, the nonwoven fabric is generally classified into a short fiber nonwoven fabric, long fiber nonwoven fabric, spunbonded nonwoven fabric, meltblown nonwoven fabric, spunlace nonwoven fabric, thermal bond (air-through) nonwoven fabric, needle punch nonwoven fabric, point bond nonwoven fabric, laminated nonwoven fabric (including SMS nonwoven fabric, SMMS nonwoven fabric, or the like having a meltblown layer sandwiched between spunbonded layers), and the like depending on a fiber length, sheet forming method, fiber bonding method, and stacked structure, and any of these nonwoven fabrics can be used.

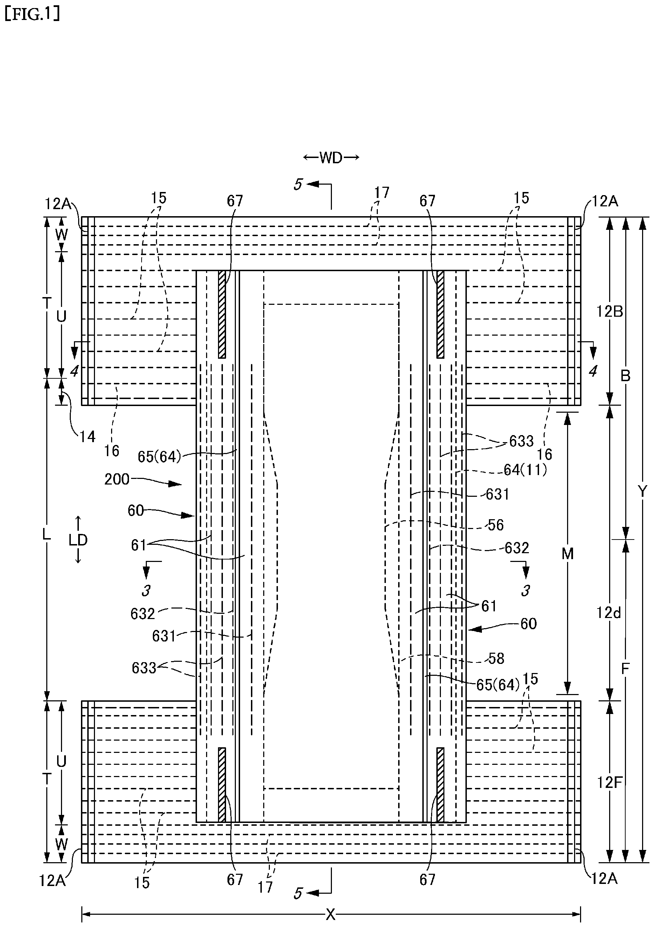

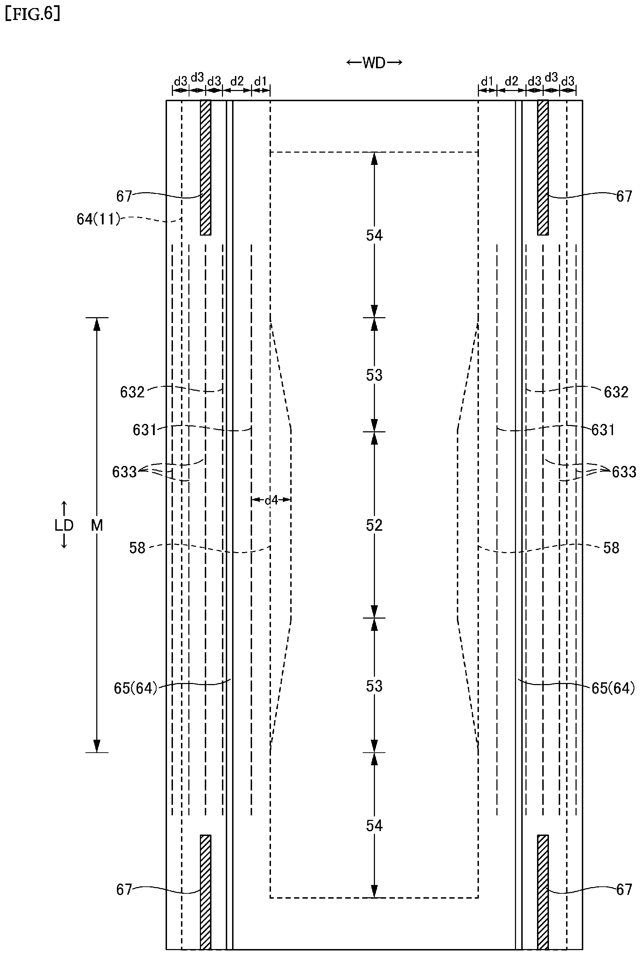

Figs. 1 to 7 illustrate an example of an underpants-type disposable diaper. The underpants-type disposable diaper 100 includes: a rectangular shaped front outer member 12F forming a lower torso portion of a front body part F; a rectangular shaped back outer member 12B forming a lower torso portion of a back body part B; and an inner member 200 provided on a middle part in a width direction WD of an outer member 12F, 12B to extend from the front outer member 12F to the back outer member 12B through a crotch part M. Both side portions of the front outer member 12F and both side portions of the back outer member 12B are bonded to each other to form side seal portions 12A, thereby forming a waist opening WO, which is formed by a front edge and a back edge of the outer member 12F, 12B and through which a wearer's lower torso passes, and leg openings LO, which are provided on both sides in the width direction WD of the inner member 200 so as to be surrounded by lower edges of the outer member 12F, 12B and side edges of the inner member 200, and through which wearer's legs pass, respectively. The inner member 200 is a portion absorbing and holding excrement e.g. urine and the like, and the outer member 12F, 12B is a portion for supporting the inner member 200 with respect to a wearer's body. A reference sign Y represents a maximum length of the diaper in a spread state (length in a front-back direction from an edge of the waist opening WO of the front body part F to an edge of the waist opening WO of the back body part B), and a reference sign X represents the maximum width of the diaper in the spread state.

The underpants-type disposable diaper 100 includes a lower torso region T formed as a range in the front-back direction having the side seal portions 12A (a range in the front-back direction from the waist opening WO to upper ends of the leg openings LO) and an intermediate region L formed as a range in the front-back direction of a portion forming the leg openings LO (between a region in the front-back direction having the side seal portions 12A of the front body part F and a region in the front-back direction having the side seal portions 12A of the back body part B). A portion located as the lower torso region T of the front outer member 12F and the lower torso region T of the back outer member 12B, namely, as the lower torso portion of the front body part F and the lower torso portion of the back body part B may be conceptually divided into a "waist end portion" W forming an edge portion of the waist opening and an "under-waist end portion" U corresponding to a portion below the waist end portion. Normally, in a case where the portion located as the lower torso portion of the front body part F and the lower torso portion of the back body part B has a boundary in which a stretching force in the width direction WD changes (for example, the fineness of elastic members or the stretch rate thereof changes), a portion closer to the waist opening WO than the boundary closest to the waist opening WO is set as the waist end portion W. In a case where there is no such a boundary, a portion, which extends toward the waist opening WO than an absorber 56 or the inner member 200, is set as the waist end portion W. The lengths in the front-back direction of the above portions vary depending on the size of a product and can be appropriately determined, but for example, the length of the waist end portion W can be 15 to 40 mm, and the length of the under-waist end portion U can be 65 to 120 mm. Meanwhile, the intermediate region L is narrowed on both side edges in substantially U shapes or curved shapes so as to follow peripheries of the wearer's legs, and both the side edges correspond to sites through which the wearer's legs are inserted. Therefore, the underpants-type disposable diaper in the spread state has a substantially hourglass shape as a whole.

### (Outer member)

As in the illustrated example, the outer member 12F, 12B is composed of the front outer member 12F, which is a rectangular shaped member forming at least the lower torso portion of the front body part F, and the back outer member 12B, which is a rectangular shaped member forming at least the lower torso portion of the back body part B, respectively. The front outer member 12F and the back outer member 12B may not be continuous on a crotch side but may be separated in the front-back direction LD (outer member separated type), or the front outer member 12F and the back outer member 12B may be continuous from the front body part F to the back body part B (outer member integrated type), although not illustrated. In an outer member separated type underpants type disposable diaper, the separation distance 12d in the front-back direction can be set to, for example, about 40 to 60% of the maximum length Y of the diaper. In the illustrated example, the lower edge of the front outer member 12F and the lower edge of the back outer member 12B may be linear shaped along the width direction WD. However, at least one of the lower edge of the front outer member 12F and the lower edge of the back outer member 12B may be curved so as to follow the peripheries of the wearer's legs.

In the outer member separated type underpants type disposable diaper, since the inner member 200 is exposed between the front outer member 12F and the back outer member 12B, in order to prevent the liquid impervious film 11 from being exposed on an underside surface of the inner member 200, a cover nonwoven fabric layer 13 is favorably provided that covers the underside surface of the inner member 200 from a space between the front outer member 12F and the inner member 200 to a space between the back outer member 12B and the inner member 200. An internal surface of the cover nonwoven fabric layer 13 and an external surface of the cover nonwoven fabric layer 13 can be bonded to respective facing surfaces by the hot melt adhesive. A nonwoven fabric used for the cover nonwoven fabric layer 13 can be selected as appropriate, and for example, a nonwoven fabric similar to the nonwoven fabric forming the outer member 12F, 12B can be selected.

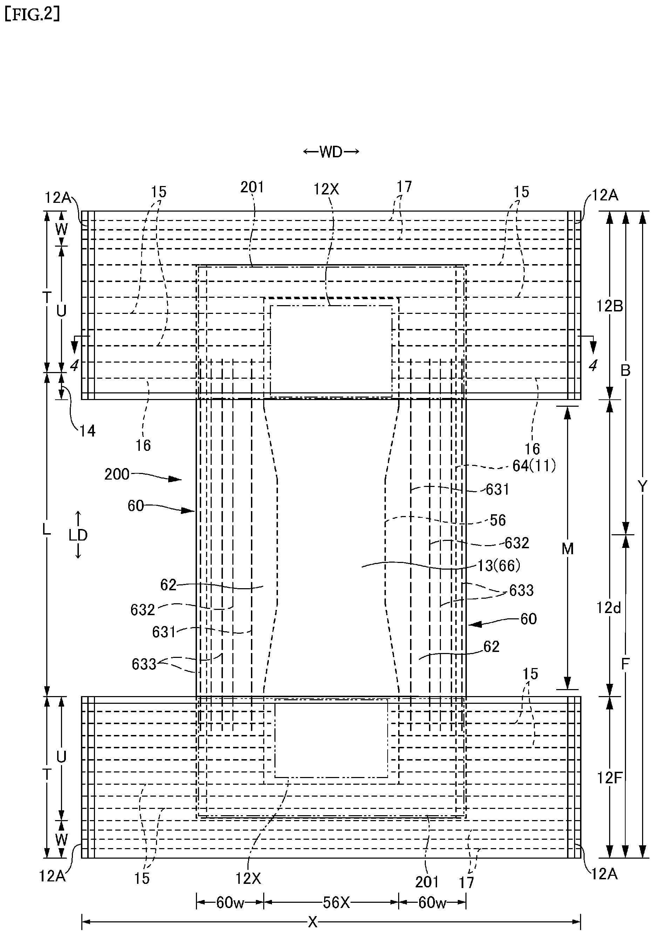

The front outer member 12F and the back outer member 12B have the lower torso portion of the front body part F and the lower torso portion of the back body part B, respectively, and these lower torso portions form the lower torso region T. In the example illustrated in Figs. 1 and 2, the back outer member 12B has a longer dimension in the front-back direction than the front outer member 12F, and the front outer member 12F does not have any part corresponding to the intermediate region L, while the back outer member 12B may have a gluteal cover portion 14 extending from the lower torso region T into the intermediate region L. However, it is also possible that a dimension in the front-back direction LD of the front outer member 12F is the same as a dimension in the front-back direction LD of the back outer member 12B and the front outer member 12F and the back outer member 12B do not have a part corresponding to the intermediate region L.

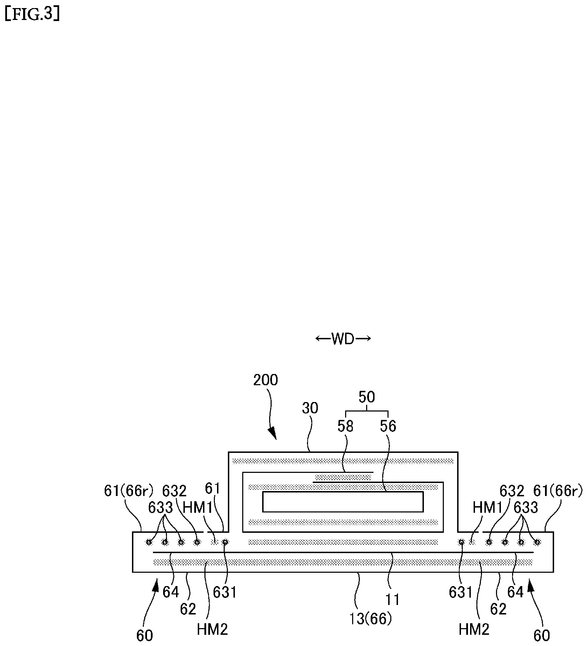

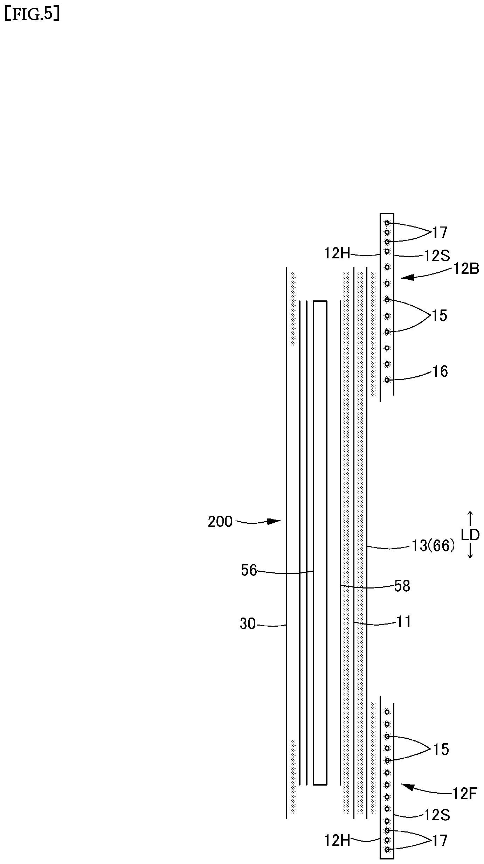

As illustrated in Figs. 3 to 5, the outer member 12F, 12B is formed by bonding an outer sheet layer 12S and an inner sheet layer 12H, which are adjacent to elastic members 15 to 17 described later on an outer side and an inner side thereof respectively, by a bonding means such as a hot melt adhesive or welding. The outer sheet layer 12S and the inner sheet layer 12H may be formed by folding back a single sheet of sheet material such that a fold line is located on a waist opening-side, as illustrated in Fig. 5. Alternatively, the outer sheet layer 12S and the inner sheet layer 12H may be formed by bonding two sheet materials to each other, although not illustrated.

For the outer sheet layer 12S and the inner sheet layer 12H, any sheet materials can be used without particular limitation, but a nonwoven fabric is favorable. In a case where the nonwoven fabric is used, a basis weight per one sheet thereof is preferably about 10 to 30 g/m².

To improve fitting of the outer member 12F, 12B to the wearer's lower torso, in the outer member 12F, 12B, the elastic members 15 to 17 are attached between the outer sheet layer 12S and the inner sheet layer 12H at a predetermined stretch rate so as to form a stretchable region which elastically stretches and contracts in the width direction WD due to stretching and contracting of the elastic members 15 to 17. In a natural length state, the stretchable region contracts due to contraction of the elastic members 15 to 17 such that wrinkles or pleats are formed, but the stretchable region can be stretched in the longitudinal direction of the elastic members 15 to 17 up to the predetermined stretch rate at which it fully extends without wrinkles. As the elastic members 15 to 17, it is possible to use a known elastic member such as a belt-shaped elastic member, net-shaped elastic member, film-shaped elastic member in addition to an elongated elastic member such as a rubber thread (illustrated example), without particular limitation. Synthetic rubber or natural rubber also may be used as the elastic members 15 to 17. For bonding the outer sheet layer 12S and the inner sheet layer 12H to each other, and for fixing the elastic members 15 to 17 interposed therebetween in the outer member 12F, 12B, it is possible to use at least one of a hot melt adhesive based on various coating methods and means based on material welding such as heat sealing, ultrasonic sealing, etc. When the entire surfaces of both the sheet layers in the outer member 12F, 12B are strongly bonded, flexibility is impaired. Thus, it is preferable that a portion other than bonded portions of the elongated elastic members 15 to 17 is not bonded or is weakly bonded. In the illustrated example, the hot melt adhesive is applied only to the outer peripheral surfaces of the elongated elastic members 15 to 17 by coating means such as a comb gun or a sure-wrap nozzle and then, the hot melt adhesive-coated elongated elastic members 15 to 17 are interposed between both the sheet layers 12S and 12H. In this way, fixing of the elongated elastic members 15 to 17 to both the sheet layers 12S and 12H as well as fixing of both the sheet layers 12S and 12H to each other are performed only with the hot melt adhesive applied to the outer peripheral surfaces of the elongated elastic members 15 to 17.

The elastic members 15 to 17 of the illustrated example will be described in more detail. Plural waist end elastic members 17 are attached between the outer sheet layer 12S and the inner sheet layer 12H in the waist end portion W of the outer member 12F, 12B at intervals in the front-back direction in a state where the waist end elastic members 17 are stretched along the width direction at a predetermined stretch rate, such that the stretchable region is formed to be continuous over a whole region in the width direction WD of the waist end portion W. In addition, out of the waist end elastic members 17, one or plural members, which are arranged in a region adjacent to the under-waist end portion U, may overlap the inner member 200, or may be provided on both respective sides in the width direction of a center portion overlapping the inner member 200 in the width direction excluding the center portion itself. As the waist end elastic members 17, it is preferable to provide about 2 to 15, particularly 4 to 10 rubber threads having a fineness of about 155 to 1,880 dtex, particularly 470 to 1,240 dtex (in the case of synthetic rubber. In the case of natural rubber, a cross-sectional area is about 0.05 to 1.5 mm², particularly 0.1 to 1.0 mm²) at intervals of 2 to 12 mm, particularly 3 to 7 mm, and it is preferable that a stretch rate in the width direction WD of the waist end portion W resulting therefrom is about 150 to 400%, particularly 220 to 320%. In the waist end portion W, all of the elastic members 17 in the front-back direction LD do not have to have the same fineness and do not have to have the same stretch rate. For example, the fineness of the elastic members 17 partly may be different each other and the stretch rate thereof partly may be different each other.

In addition, plural under-waist end elastic members 15 formed of elongated elastic members are attached between the outer sheet layer 12S and the inner sheet layer 12H in the under-waist end portion U of the outer member 12F, 12B, on both respective sides in the width direction, at intervals in the front-back direction in a state where the under-waist end elastic members 15 are stretched along the width direction at a predetermined stretch rate, such that the stretchable region is formed on both the respective sides in the width direction WD excluding the center portion overlapping the inner member 200. As the under-waist end elastic members 15, it is preferable to dispose about 5 to 30 rubber threads each having a fineness of about 155 to 1880 dtex, particularly 470 to 1240 dtex (in the case of synthetic rubber. In the case of natural rubber, a cross-sectional area is about 0.05 to 1.5 mm², particularly 0.1 to 1.0 mm²) at intervals of 1 to 15 mm, particularly 3 to 8 mm, and it is preferable that a stretch rate in the width direction WD of the under-waist end portion U resulting therefrom is about 200 to 350%, particularly 240 to 300%. In the under-waist end portion U, all of the elastic members 15 in the front-back direction LD do not have to have the same fineness and do not have to have the same stretch rate, and the fineness of the elastic members 15 partly may be different each other and the stretch rate thereof partly may be different each other.

Further, one or plural gluteal cover elastic members 16 formed of elongated elastic members are attached between the outer sheet layer 12S and the inner sheet layer 12H in the gluteal cover portion 14 of the back outer member 12B, on both respective sides in the width direction of a center portion overlapping the inner member 200 in the width direction excluding the center portion itself, at intervals in the front-back direction in a state where the gluteal cover elastic members 16 are stretched along the width direction at a predetermined stretch rate, such that the stretchable region is formed on both the respective sides in the width direction WD of the center portion overlapping the inner member 200 in the width direction excluding the center portion itself. As the gluteal cover elastic members 16, it is preferable to fix about 2 to 10 rubber threads each having a fineness of about 155 to 1880 dtex, particularly 470 to 1240 dtex (in the case of synthetic rubber. In the case of natural rubber, a cross-sectional area is about 0.05 to 1.5 mm², particularly 0.1 to 1.0 mm²) at intervals of 5 to 40 mm, particularly 5 to 20 mm, at a stretch rate of 150 to 300%, particularly 180 to 260%.

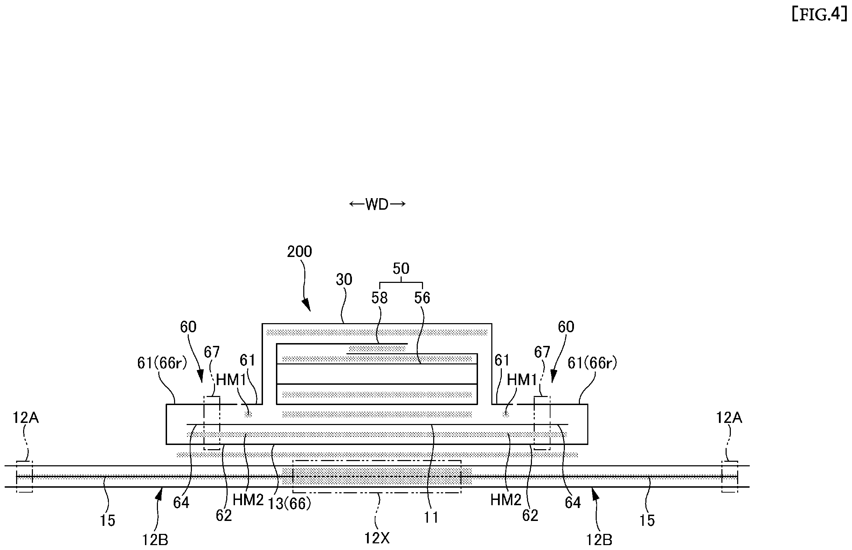

Incidentally, as in the illustrated example, when the under-waist end elastic members 15 and the gluteal cover elastic members 16 are disposed on both respective sides of a part or whole of a portion overlapping with the inner member 200 in the width direction WD excluding the part or the whole of the portion itself, the inner member 200 is not contracted more than necessary in the width direction, and a poor appearance such as a lumpy appearance is prevented, and an absorption performance is not reduced. In such configuration, in addition to a case in which the elastic members 15, 16 exist only on both the sides of the portion overlapping with the inner member 200 in the width direction, a case is also included in which the elastic members 15, 16 exist from one side to the other side in the width direction of the under-waist end portion U and the gluteal cover portion 14 through the portion overlapping with the inner member 200, but in a middle part or whole of the portion overlapping with the inner member 200, as represented by a reference sign 12X in Figs. 2 and 4, the elastic members 15, 16 are finely cut such that any contraction force is not applied (substantially equal to a state being free from elastic members) there, which means that the contraction force is applied only to both the respective sides of the middle part or whole of the portion overlapping with the inner member 200. It is needless to say that the arrangement of the under-waist end elastic members 15 and the gluteal cover elastic members 16 is not limited to the above examples. Precisely, some or all of the under-waist end elastic members 15 and the gluteal cover elastic members 16 may be provided from one side to the other side in the width direction of the under-waist end portion U and the gluteal cover portion 14 through the portion overlapping with the inner member 200 such that a stretching force is applied over a whole region in the width direction thereof including the portion overlapping with the inner member 200.

### (Inner Member Bonding Portion)

The inner member 200 may be fixed to the outer member 12F, 12B by bonding means based on material welding such as heat sealing or ultrasonic sealing or with a hot melt adhesive. In the illustrated example, the underside surface of the inner member 200 is fixed to an internal surface of the outer member 12F, 12B with a hot melt adhesive. In this way, each of inner member bonding portions 201 fixes the inner member 200 and each of the front outer member 12F and back outer member 12B to each other. Each of the inner member bonding portions 201 may be provided almost entirely in an overlapping region in which the inner member 200 and each of the front outer member 12F and back outer member 12B overlap with each other, as illustrated in Fig. 2. For example, as each of the inner member bonding portions 201, both end portions in the width direction of the inner member 200 are excluded from the overlapping region.

### (Inner member)

An arbitrary shape can be adopted for the inner member 200, and a rectangular shape is adopted in an illustrated example. As illustrated in Figs. 3 to 5, the inner member 200 includes a liquid pervious top sheet 30 corresponding to a wearer's body side, a liquid impervious film 11, and an absorbent element 50 interposed therebetween. Reference sign 60 denotes each side flap having a planar gather part.

### (Top sheet)

As the top sheet 30, a liquid pervious material such as a perforated or unperforated nonwoven fabric or a perforated plastic sheet can be used without particular limitation, but in a case where the top sheet 30 also serves as a cover material of a liquid impervious film 64 as illustrated in Figs. 3 and 4, a nonwoven fabric is preferably used.

In a case where both sides in the width direction WD of the top sheet 30 do not also serve as the cover material of the liquid impervious film 64, both side portions of the top sheet 30 may be folded back to an underside of the absorbent element at side edges thereof and then, extended so as to be inserted between the absorbent element 50 and the liquid impervious film 11.

### (Intermediate sheet)

To promptly transfer a liquid that has permeated through the top sheet 30 to the absorber, it is possible to provide an intermediate sheet (also referred to as the "second sheet") having a higher liquid permeation rate than that of the top sheet 30, although not illustrated. The intermediate sheet is used in order to promptly transfer a liquid to the absorber to enhance absorption performance of the absorber, and to prevent a "returning" phenomenon of the absorbed liquid from the absorber. The intermediate sheet 40 can be omitted.

### (Liquid impervious film)

A material of the liquid impervious film 11 provided on an underside of the absorber 56 is not particularly limited, but examples thereof may include a plastic film made of a polyolefin-based resin such as polyethylene, polypropylene, etc. A material having liquid impermeability and moisture permeability, which has been favorably used from the viewpoint of prevention of stuffiness, is preferably used for the liquid impervious film 11. A microporous plastic film, which is obtained by kneading an inorganic filler in a polyolefin-based resin such as polyethylene or polypropylene, molding the kneaded mixture into a sheet, and stretching the sheet in a monoaxial or biaxial direction, is widely used as the plastic film having moisture permeability.

To enhance a leakage prevention-property of the planar gather part, the liquid impervious film 11 may be configured to extend into the side flaps, as the example illustrated in Figs. 3 and 4. Alternatively, the liquid impervious film 11 may configured to have a width that fits on the underside of the absorbent element 50, the liquid impervious film 11 may fold back at the side edges of the absorbent element 50 toward a top side and then extend inwardly between the absorbent element 50 and the top sheet 30, or the like, although not illustrated.

### (Absorbent element)

As the example illustrated in Figs. 3, 4 and 9, the absorbent element 50 includes the absorber 56 and the wrapping sheet 58 wrapping the entire absorber 56. The wrapping sheet 58 may be omitted.

### (Absorber)

The absorber 56 can be formed by an assembly of fibers. As this fiber assembly, it is possible to use one obtained by accumulating short fibers of fluff pulp, synthetic fibers, etc., and in addition, a filament assembly obtained by opening a tow (fiber bundle) of synthetic fibers such as cellulose acetate as necessary. A fiber basis weight may be set to, for example, about 100 to 300 g/m² in the case of accumulating fluff pulp or short fibers, and may be set to, for example, about 30 to 120 g/m² in the case of the filament assembly. In the case of a synthetic fiber, the fineness is, for example, 1 to 16 dtex, preferably 1 to 10 dtex, more preferably 1 to 5 dtex.

It is preferable that super absorbent polymer particles may be contained in the fiber assembly. The super absorbent polymer particles include "powder" in addition to "particles". As the super absorbent polymer particles, those used for this type of disposable diaper may be used as they are. For example, it is desirable that the proportion of particles remaining on the sieve is 30 wt% or less by sieving (shaking for 5 minutes) using a 500 um standard sieve (JIS Z 8801-1: 2006), and it is desirable that the proportion of particles remaining on the sieve is 60 wt% or more by sieving (shaking for 5 minutes) using a 180 um standard sieve (JIS Z 8801-1: 2006).

As the super absorbent polymer particles, any material can be used without particular limitation, but those having a water absorption capacity (JIS K7223-1996 "Testing method for water absorption capacity of super absorbent polymers") of 40 g/g or more are suitable. Examples of the super absorbent polymer particles include a starch-based material, cellulose-based material, and synthetic polymer-based material. A starch-acrylic acid (salt) graft copolymer, saponified product of a starch-acrylonitrile copolymer, cross-linked product of sodium carboxymethyl cellulose, acrylic acid (salt) polymer, or the like can be used. As the shape of the super absorbent polymer particles, a usually used particulate material shape is suitable, but other shapes may also be used.

As the super absorbent polymer particles, for example, those having a water absorption speed of 70 seconds or less, particularly 40 seconds or less are suitably used. When the water absorption speed is too low, so-called returning, in which a liquid that has been supplied into the absorber 56 returns out of the absorber 56, tends to occur.

In addition, as the super absorbent polymer particles, those having a gel strength of 1000 Pa or more are preferably used. This makes it possible to effectively suppress a sticky feeling after liquid absorption, even in a case where the absorber 56 is made bulky.

A basis weight amount of the super absorbent polymer particles may be appropriately determined according to an absorption amount required for the use of the absorber 56. Therefore, although it cannot be said unconditionally, the basis weight amount may be set to 50 to 350 g/m². The basis weight of the polymer particles of less than 50 g/m² makes it difficult to secure the absorption amount. The basis weight of more than 350 g/m² saturates an effect.

A ratio of fibers to super absorbent polymer particles in the absorber 56 is not particularly limited. However, when a weight ratio of fibers : super absorbent polymer particles corresponds to 50 : 50 to 20 : 80, and when comparison is performed at the same area and the same absorption amount, the thinner absorber 56 may be obtained. In this case, a thickness 56t of the absorber 56 is not particularly limited. However, the thickness 56t may be set to 3 to 15 mm.

The absorber 56 is provided to be continuous from a position on the front side of the crotch part M to a position on the back side of the crotch part M. In the illustrated example, it is preferable that the absorber 56 is provided to be continuous from the front outer member 12F to the back outer member 12B. Note that a reference sign 56X designates the maximum width of the absorber 56.

For ease of securing an absorption amount in the crotch part M, it is preferable that the absorber 56 is formed into a substantially rectangular shape. On the other hand, it is also preferable that, as illustrated in Figs 1 and 2, the absorber 56 has a portion 52 with a minimum width in the crotch part M, and a widening portion 53, which is provided between the portion 52 with the minimum width and a portion 54 with a maximum width on the front side of the crotch part M so as to widen forward gradually, and a widening portion 53, which is provided between the portion 52 with the minimum width and a portion 54 with the maximum width on the back side of the crotch part M so as to widen backward gradually, because this configuration of the absorber makes it possible that the crotch part M fits well to wearer's inner thighs due to a narrower portion 52, 53 formed of the portion 52 with the minimum width and the widening portions 53. Further, also in this case, if ease of securing the absorption amount in the crotch part M is required, it is preferable that in the crotch part M, the minimum width 52w of the absorber 56 is equal to or more than 0.8 times the maximum width 56X of the absorber 56.

In addition, a dimension in the front-back direction of the narrower portion 52, 53 is preferably about 20 to 30% of the maximum length Y of the product.

Incidentally, in a case where the absorber 56 has the above mentioned narrower portion 52, 53, the crotch part M refers to a range in the front-back direction LD having the narrower portion 52, 53; in a case where the absorber 56 does not have the narrower portion 52, 53, and instead, an outer shape of the diaper in the spread state has a narrower portion, the crotch part M refers to a range in the front-back direction LD having this narrower portion of the outer shape (for example, the crotch part M is disposed between the front outer member 12F and the back outer member 12B); and in a case where the absorber 56 has neither of such narrower portions, the crotch part M refers to a part disposed at a center in the front-back direction LD and a dimension in the front-back direction LD thereof is 20 to 30% of a maximum length Y of the product.

### (Wrapping sheet)

When the diaper includes the wrapping sheet 58, as the material thereof, a liquid pervious material such as tissue paper, in particular, crepe paper, a nonwoven fabric, polyethylene-laminated nonwoven fabric, perforated sheet, or the like can be used, and sheets through which the super absorbent polymer particles will not escape are preferred. When a nonwoven fabric is used in place of crepe paper, a hydrophilic SMS (SMS, SSMMS, or the like) nonwoven fabric is particularly preferred, which may be made of polypropylene, a polyethylene/polypropylene composite material, or the like. The basis weight of the wrapping sheet 58 is preferably 5 to 40 g/m², particularly 10 to 30 g/m².

A wrapping mode with the wrapping sheet 58 may be determined appropriately, and in view of ease of manufacturing and in view of limiting escape of the super absorbent polymer particles from front and back end edges of the wrapping sheet 58, the following mode is preferable. Precisely, the wrapping sheet 58 winds around the absorber 56 in a tubular shape so as to surround a top surface, an underside surface, and both side surfaces thereof, while a front end edge portion and a back end edge portion of the wrapping sheet 58 are made to protrude forward and backward from the absorber 56, respectively, and each of these protruded portions is pressed to be flat in a top side-underside direction such that two sheets thereof are bonded to each other by bonding means such as a hot melt adhesive.

### (Side flap)

As illustrated in Figs. 1 to 4 and 6, a pair of side flaps 60 are provided to project laterally from both side edges of the absorber 56 on both sides in the width direction WD of the absorber 56 and extend along the front-back direction LD from a front end portion of the inner member 200 to a back end portion thereof, respectively. Characteristically, each side flap 60 includes a planar gather part provided to be continuous from a lateral side of the portion 54 with the maximum width of the absorber 56 on the front side of the crotch part M to a lateral side of the portion 54 with the maximum width of the absorber 56 on the back side of the crotch part M. The planar gather part has elongated gather elastic members 631 to 633, which are incorporated therein along the front-back direction LD such that the planar gather part is contracted in the front-back direction LD due to contraction of the gather elastic members 631 to 633 and stretchable in the front-back direction LD due to stretching of the gather elastic members 631 to 633. In addition, the underpants-type disposable diaper is free of three-dimensional gather parts rising toward the top side, and has only the planar gather parts of the side flaps 60.

To suppress an influence exerted on a side portion of the absorber 56 by a leg movement, each side flap 60 is desirably secured to have a sufficiently large width. Therefore, the side flap 60 has a width 60w being preferably 0.1 to 0.5 times, more preferably 0.3 to 0.5 times, and in particular preferably 0.4 to 0.5 times the maximum width 56X of the absorber 56. Additionally, it is desirable that the gather elastic members 631 to 633 are composed of a first gather elastic member 631 attached on a lateral side of side edges of the portions 54 with the maximum width of the absorber 56 such that there is a first distance d1 between the first gather elastic member 631 and the side edges of the portions 54 with the maximum width, a second gather elastic member 632 attached on a lateral side of the first gather elastic member 631 such that there is a second distance d2 between the second gather elastic member 632 and the first gather elastic member 631, and third gather elastic members 633 attached repeatedly to a side edge portion of the side flap 60 on a lateral side of the second gather elastic member 632 such that there is a third distance d3 for each space between two adjacent members of the second gather elastic member 632 and the third gather elastic members 633 (That is, there are no other elastic members than these gather elastic members 631 to 633), the second distance d2 is 1.5 to 5 times the third distance d3, and the first distance d1 is 0.3 to 0.8 times the second distance d2. It is more desirable that the second distance d2 is 1.5 to 2.5 times the third distance d3, and the first distance d1 is 0.3 to 0.7 times the second distance d2. More specifically, in a case of a diaper for babies and infants, the side flap 60 has preferably a width being 30 to 50 mm, in particular preferably 40 to 50 mm.

Further, on the basis that the first gather elastic member 631 has a first fineness and is attached to the side flap 60 at a first stretch rate; the second gather elastic member 632 has a second fineness and is attached to the side flap 60 at a second stretch rate; and each of the third gather elastic members 633 has a third fineness and is attached to the side flap 60 at a third stretch rate, it is desirable that
(a1) the first stretch rate is 0.95 to 1.05 times the second stretch rate and is 0.95 to 1.05 times the third stretch rate and at the same time, the first fineness is 1.2 to 1.5 times the second fineness and is 1.2 to 1.5 times the third fineness,
(b1) the first fineness is 0.95 to 1.05 times the second fineness and is 0.95 to 1.05 times the third fineness and at the same time, the first stretch rate is 1.2 to 1.5 times the second stretch rate and is 1.2 to 1.5 times the third stretch rate, or
(c1) the first fineness is 1.2 to 1.5 times the second fineness and is 1.2 to 1.5 times the third fineness, and at the same time, the first stretch rate is 1.2 to 1.5 times the second stretch rate and is 1.2 to 1.5 times the third stretch rate.

Further, it is more desirable that
(a2) the first stretch rate is 0.95 to 1.05 times the second stretch rate and is 0.95 to 1.05 times the third stretch rate, and at the same time, the first fineness is 1.3 to 1.4 times the second fineness and is 1.3 to 1.4 times the third fineness,
(b2) the first fineness is 0.95 to 1.05 times the second fineness and is 0.95 to 1.05 times the third fineness, and at the same time, the first stretch rate is 1.3 to 1.5 times the second stretch rate and is 1.3 to 1.5 times the third stretch rate, or
(c2) the first fineness is 1.3 to 1.4 times the second fineness and is 1.3 to 1.4 times the third fineness, and at the same time, the first stretch rate is 1.3 to 1.5 times the second stretch rate and is 1.3 to 1.5 times the third stretch rate.

As the gather elastic members 631 to 633, it is possible to use an elongated elastic member such as a thread-shaped rubber and belt-shaped rubber. In a case where the rubber thread is used, the first to third fineness is preferably 400 to 950 dtx, more preferably 470 to 780 dtx, in addition, the first to third stretch rate is preferably 200 to 320%, more preferably 260 to 320%.

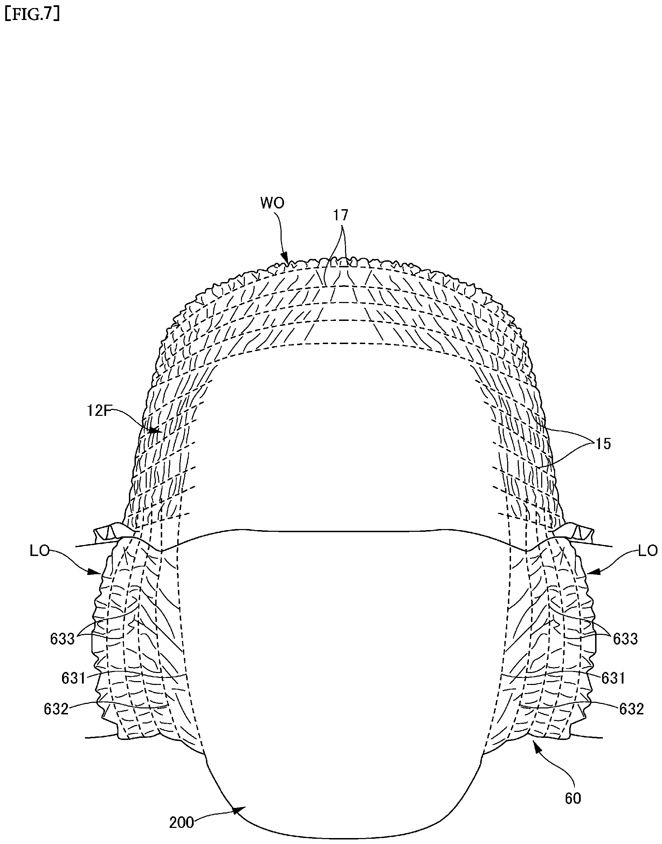

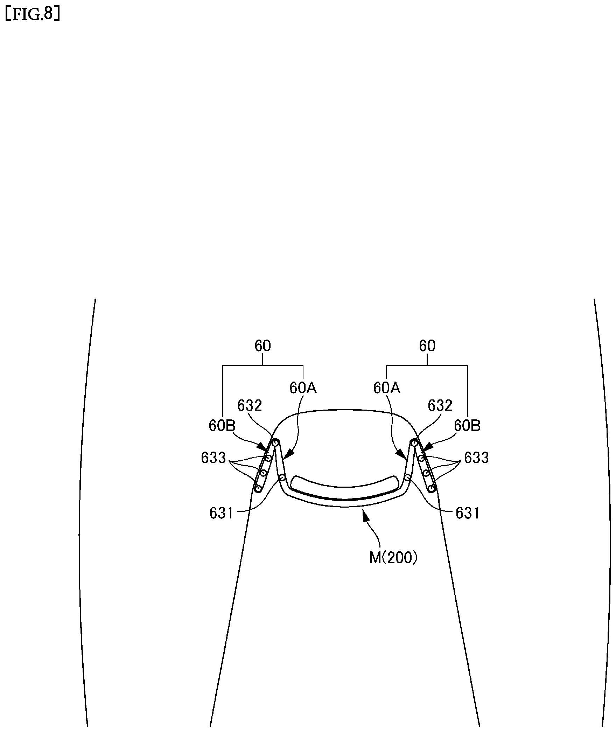

As stated above, the side flap 60 including the planar gather part is secured to have a sufficiently large width, and additionally, the first gather elastic member 631 is provided near the absorber 56, the second gather elastic member 632 is provided at a position which is laterally separated from the first gather elastic member 631 to some degree, and the third gather elastic members 633 are provided repeatedly to the side edge portion of the side flap 60 at short intervals. In addition, a force required for stretching the first gather elastic member 631 provided near the absorber 56 is stronger than a force required for stretching the second gather elastic member 632 and is stronger than a force required for stretching the third gather elastic member 633. As a result, in an appropriate worn state, the situation as illustrated in Figs. 7 and 8 is caused. Precisely, in the crotch part M, a portion, which is located between the side edge of the absorber 56 and the second gather elastic member 632, extends obliquely in an upward direction toward a root of a wearer's inner thigh as a first portion 60A, and a portion, which is located between the second gather elastic member 632 and a side edge of the side flap 60, extends obliquely along the wearer's inner thigh in a downward direction as a second portion 60B. Then, the first portion 60A is raised due to contraction of the first gather elastic member 631 by a stronger force than a force by which the second portion 60B is raised, such that the first portion 60A is unlikely to move. Thus, the second portion 60B, which is located at the position separated from the first gather elastic member 631 to some degree, is brought closely into contact with the wearer's inner thigh, while the second portion 60B is supported by the first gather elastic member 631. Therefore, even if the second portion 60B moves according to a leg movement, since a section, which is located between the first gather elastic member 631 and the second gather elastic member 632, is deformed so as to have a buffer effect, it is unlikely to happen that the movement of the second portion 60B is transmitted to the absorber 56 through the first portion 60A, which decreases a possibility of loss of shape in the side portion of the absorber 56.

As in the illustrated example, in a case where the absorber 56 has the portion 52 with the minimum width in the crotch part M, the widening portion 53, which is provided between the portion 52 with the minimum width and the portion 54 with the maximum width on the front side so as to widen forward gradually, and the widening portion 53, which is provided between the portion 52 with the minimum width and the portion 54 with the maximum width on the back side so as to widen backward gradually, if a distance in the width direction WD between a side edge of the portion 52 with the minimum width of the absorber 56 and the first gather elastic member 631 is too large, a portion, which is located between the side edge of the portion 52 with the minimum width of the absorber 56 and the first gather elastic member 631, tends to be concaved deeply to an underside such that excrement may remain on the side flap 60, a finger of the wearer's leg may be caught on the portion when the wearer puts on the diaper or the like. The distance d4 in the width direction WD between the side edge of the portion 52 with the minimum width of the absorber 56 and the first gather elastic member 631 is preferably 1.1 to 1.5 times, particularly 1.1 to 1.3 times the second distance d2.

A configuration of the side flap 60 can be determined appropriately and it is possible to adopt a similar configuration to those of Patent Literature 1 to 3, but the configuration of the illustrated example is preferable, because it is simple and excellent in view of waterproof property and ease of manufacturing. Precisely, the side flap 60 in the illustrated example includes a top surface-nonwoven fabric layer 61 forming a top surface of the side flap 60, an underside surface-nonwoven fabric layer 62 forming an underside surface of the side flap 60, the elongated gather elastic members 631 to 633 provided between the top surface-nonwoven fabric layer 61 and the underside surface-nonwoven fabric layer 62 to extend along the front-back direction and a liquid impervious film 64(11) extending over a range from a base to a position closer to a tip than the base and sandwiched between the top surface-nonwoven fabric layer 61 and the underside surface-nonwoven fabric layer 62. In the illustrated example, a nonwoven fabric-absent portion 65 in which the top surface-nonwoven fabric layer 61 is absent and the liquid impervious film 64 is exposed is formed in the width direction of the side flap. However, the top surface-nonwoven fabric 61 may extend over almost a whole region in the width direction of the side flap. In a case where the nonwoven fabric-absent portion 65 is provided, the width thereof may be determined appropriately, but is preferably 10 mm or less.

Specifically, it is preferable that the section, which is located between the first gather elastic member 631 and the second gather elastic member 632, has the portion 65 in which the top surface-nonwoven fabric layer 61 is absent and the liquid impervious film 64 is exposed (in the top surface-nonwoven fabric layer 61, even a cut line having substantially no width can be referred to as the portion 65), because rigidity of this section becomes low such that the section can be particularly deformable.

Since the section, which is located between the first gather elastic member 631 and the second gather elastic member 632, has the buffer effect, it is preferable that this section has low rigidity. As the illustrated example, in a case where the section, which is located between the first gather elastic member 631 and the second gather elastic member 632, has a three layered structure formed of the top surface-nonwoven fabric layer 61, the underside surface-nonwoven fabric layer 62, and the liquid impervious film disposed therebetween, the top surface-nonwoven fabric layer 61 and the liquid impervious film 11 are bonded to each other through a first hot melt adhesive HM1, and the underside surface-nonwoven fabric layer 62 and the liquid impervious film 11 are bonded to each other through a second hot melt adhesive HM2, each of the top surface-nonwoven fabric layer 61 and the underside surface-nonwoven fabric layer 62 has preferably a bending resistance of 0.2 to 2.2 mN.cm in the front-back direction LD and 0.05 to 0.5 mN.cm in the width direction WD, in particular preferably a bending resistance of 0.4 to 0.6 mN.cm in the front-back direction LD and 0.07 to 0.1 mN.cm in the width direction WD, according to 41.5° Cantilever Method defined by JIS L 1913: 2010. Further, the liquid impervious film has preferably a bending resistance of 0.006 to 0.05 mN.cm in the front-back direction LD and 0.006 to 0.05 mN.cm in the width direction WD, in particular preferably a bending resistance of 0.006 to 0.02 mN.cm in the front-back direction LD and 0.006 to 0.02 mN.cm in the width direction WD, according to 41.5° Cantilever Method defined by JIS L 1913: 2010. Furthermore, an application amount of the first hot melt adhesive HM1 is preferably 1 to 10 g/m², in particular preferably 1 to 5 g/m², and an application amount of the second hot melt adhesive HM2 is preferably 1 to 10 g/m², in particular preferably 1 to 5 g/m².

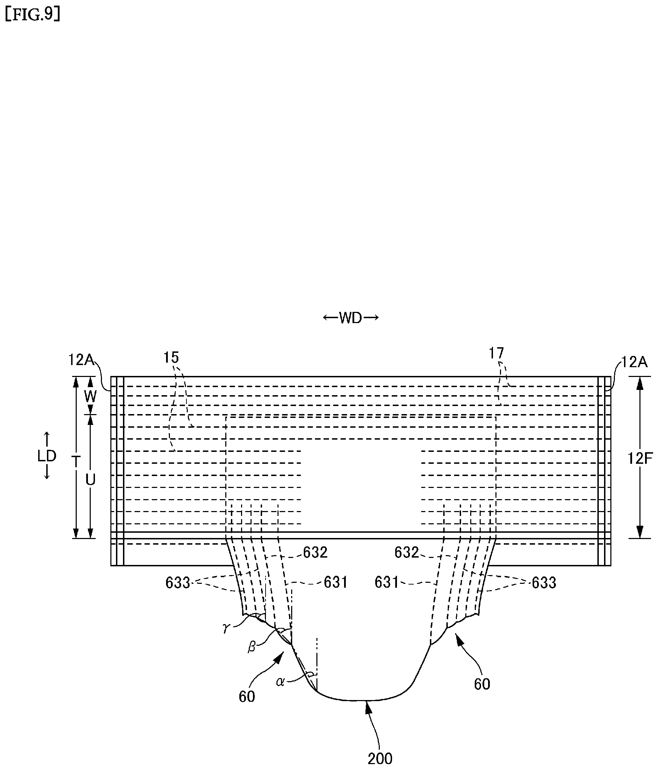

In addition, as illustrated in Fig. 9, when the underpants-type disposable wearing article is placed on a horizontal plane while the back body part B is located on a bottom side and only the outer member 12F, 12B is stretched from a natural length state to a spread state along the horizontal plane for viewing the underpants-type disposable wearing article from above, it is preferable that in an outline of the crotch part M, an acute intersecting angle α between the front-back direction LD and an imaginary line connecting a position of the side edge of the absorber 56 and a position of the first gather elastic member 631 is 15 to 40 degrees, an acute intersecting angle γ between the front-back direction LD and an imaginary line connecting two positions of the third gather elastic members 633 adjacent to each other is 50 to 90 degrees, and an acute intersecting angle β between the front-back direction LD and an imaginary line connecting the position of the first gather elastic member 631 and a position of the second gather elastic member 632 is larger than α and is smaller than γ, because the buffer effect explained before becomes more excellent. Angle α is more preferably 25 to 35 degrees. Angle γ is more preferably 55 to 70 degrees. Angle β is more preferably about 1.3 to 1.7 times angle α.

In the side flap 60, a region in a front-back direction LD where the gather elastic members 631 to 633 are provided can be determined appropriately, as long as the range is provided to be continuous from a lateral side of the portion 54 with the maximum width of the absorber 56 on the front side of the crotch part M to a lateral side of the portion 54 with the maximum width of the absorber 56 on the back side of the crotch part M. However, it is desirable that the range is provided to be continuous from the stretchable region of the lower torso region T in the front body part F to the stretchable region of the lower torso region T in the back body part B.

As long as the gather elastic members 631 to 633 are provided between the top surface-nonwoven fabric layer 61 and the underside surface-nonwoven fabric layer 62 (accordingly, the gather elastic members 631 to 633 cannot be provided in the nonwoven fabric-absent portion 65), the gather elastic members 631 to 633 can be provided on either a top side of the liquid impervious film 64 contained in the side flap 60 as illustrated in Fig. 3, or an underside thereof although not illustrated.

An arrangement of the liquid impervious film 64 in the side flap 60 can be determined appropriately. If the waterproof property is important, it is preferable that the liquid impervious film 64 extends over a whole region in the width direction of the side flap. However, to secure flexibility of the side edge of the side flap, it is preferable that the liquid impervious film 64 is not provided in the side edge portion of the side flap (the side edge portion is, for example, a portion extending from a position between an outermost third gather elastic member and a next third gather elastic member adjacent thereto to the side edge of the side flap).

The top surface-nonwoven fabric layer 61 and the underside surface-nonwoven fabric layer 62 may be formed of nonwoven fabrics, which are different from those of the top sheet 30 and the cover nonwoven fabric layer 13, but these layers 61, 62 are preferably formed of nonwoven fabrics, which are common with those of the top sheet 30 and the cover nonwoven fabric layer 13. Precisely, in the illustrated example, the top sheet 30 is formed of a nonwoven fabric, and both sides in the width direction WD of the top sheet 30 are configured to extend laterally from the side edges of the absorber 56. Then, a nonwoven fabric 66 forming the cover nonwoven fabric layer 13 is disposed on the underside of the absorber 56, and both sides in the width direction WD of the nonwoven fabric 66 are configured to extend laterally from the side edges of the absorber 56. Furthermore, both side end portions of the nonwoven fabric 66 are folded back inwardly, and tips of both folded-back portions 66r are separated from tips of the top sheet 30 on both sides in the width direction WD, respectively. In addition, the liquid impervious film 64 is disposed in a region covering at least from an inside-position of each one of the folded-back portions 66r to a position between the top sheet 30 and the nonwoven fabric 66. As a result, the underside surface-nonwoven fabric layer 62 is formed by a portion other than the folded-back portions 66r of the nonwoven fabric 66, while the top surface-nonwoven fabric layers 61 are formed by the folded-back portions 66r of the nonwoven fabric 66 and portions of the top sheet 30 extending to both lateral sides of the absorber 56. Further, the nonwoven fabric-absent portions 65 are formed by portions in each of which the folded-back portion 66r of the nonwoven fabric 66 and the top sheet 30 are separated from each other. In this way, in both the side flaps 60, portions of the top surface-nonwoven fabric layers 61, which are closer to the absorber 56 than the nonwoven fabric-absent portions 65, are formed by the top sheet 30, and the other portions of the top surface-nonwoven fabric layers 61 are formed by the folded-back portions 66r of the nonwoven fabrics 66, respectively. Therefore, the nonwoven fabric-absent portions 65 can be provided without cutting a material, and additionally, a very simple structure and ease of manufacturing can be attained.

Here, it is preferable that the liquid impervious film 64 contained in the side flaps 60 extends from one of the side flaps 60 to the other of the side flaps 60 through the underside of the absorber 56 as illustrated in Figs. 3 and 4, because not only a water shielding property of the side flaps 60 but also a water shielding property of the underside of the absorber 56 can be secured at the same time. However, the liquid pervious film 64 contained in the side flaps 60 and the liquid pervious film 11 covering the underside of the absorber 56 can be disposed separately from each other. In this case, a material of the liquid impervious film 64 contained in the side flaps 60 may be the same as or may be different from(, for example, may have a lower rigidity than) a material of the liquid impervious film 11 covering the underside of the absorber 56.

For bonding the top surface-nonwoven fabric layer 61 and the underside surface-nonwoven fabric layer 62 to each other, and for fixing the gather elastic members 631 to 633 interposed therebetween, it is possible to use at least one of a hot melt adhesive based on various coating methods and means based on material welding such as heat sealing, ultrasonic sealing, etc. When the entire surfaces of the top surface-nonwoven fabric layer 61 and the underside surface-nonwoven fabric layer 62 are bonded, flexibility is impaired. Thus, it is preferable that a portion other than a bonded portion of the gather elastic members 631 to 633 is not bonded or is weakly bonded. In the illustrated example, the hot melt adhesive is applied only to the outer peripheral surfaces of the gather elastic members 631 to 633 by coating means such as a comb gun or a sure-wrap nozzle and then, the hot melt adhesive-coated gather elastic members 631 to 633 are interposed between the top surface-nonwoven fabric layer 61 and the underside surface-nonwoven fabric layer 62. In this way, fixing of the gather elastic members 631 to 633 to the top surface-nonwoven fabric layer 61 and to the underside surface-nonwoven fabric layer 62 as well as fixing of the top surface-nonwoven fabric layer 61 and the underside surface-nonwoven fabric layer 62 are performed only with the hot melt adhesive applied to the outer peripheral surface of the gather elastic members 631 to 633. As each end portion of the top sheet 30 in the illustrated example, in an end portion of a nonwoven fabric or sheet which is separated from any of the gather elastic members 631 to 633, it is possible to use another hotmelt adhesive (the first hotmelt adhesive HM1 in the illustrated example) for fixing the site.

In addition, there is an area having no gather elastic members 631 to 633 in each of front and back end portions of the side flap 60. In this area, it is preferable that a member (the top surface-nonwoven fabric layer 61 in the illustrated example) provided on a top side of and adjacent to the gather elastic members 631 to 633 in the crotch part M and a member (the liquid impervious film 64 in the illustrated example) provided on an underside of and adjacent to the gather elastic members 631 to 633 in the crotch part M are fixed to each other by at least one of a hot melt adhesive and material welding (heat sealing, ultrasonic sealing, etc.) In the drawings, a fixed portion thus formed is denoted by reference sign 67.

As the top surface-nonwoven fabric layer 61 and the underside surface-nonwoven fabric layer 62, a nonwoven fabric, which is flexible and excellent in uniformity/concealing property such as a spunbonded nonwoven fabric (SS, SSS, etc.), SMS nonwoven fabric (SMS, SSMMS, etc.), meltblown nonwoven fabric, etc., can be used preferably, and as necessary, such nonwoven fabric can be made water-repellent using silicone or the like. The top surface-nonwoven fabric layer 61 and the underside surface-nonwoven fabric layer 62 have preferably a fiber basis weight of about 10 to 30 g/m². In the example illustrated in Figs. 3 and 4, as can be seen from the fact that the portion of the top surface-nonwoven fabric layer 61, which is closer to a base than the nonwoven fabric-absent portion 65, is formed by the top sheet 30, it is possible to make a material of each of the top surface-nonwoven fabric layer 61 and the underside surface-nonwoven fabric layer 62 partially different, or it is also possible to make the materials of the top surface-nonwoven fabric layer 61 and the underside surface-nonwoven fabric layer 62 different from each other.

### <Description of Terms Used in Specification>

The following terms used in the specification should be understood to have the meanings defined below unless otherwise specified in the specification.
- "Front-back direction" means a direction (longitudinal direction) indicated by a reference sign LD in the drawing, "width direction" means a direction (left-right direction) indicated by a reference sign WD in the drawing, and the front-back direction and the width direction are orthogonal to each other.
- "Top side" means a side closer to wearer's skin when a wearing article is worn. "Underside" means a side far from wearer's skin when a wearing article is worn.
- "Top surface" means a surface of a member closer to wearer's skin when a wearing article is worn. "Underside surface" means a surface far from wearer's skin when a wearing article is worn.
- "Stretch rate" means a value obtained when a natural length is set to 100%. For example, the stretch rate of 200% has the same meaning as the elongation ratio of 2.
- "Gel strength" is measured as follows. 1.0 g of super absorbent polymer is added to 49.0 g of artificial urine (prepared by mixing 2wt% of urea, 0.8wt% of sodium chloride, 0.03wt% of calcium chloride dihydrate, 0.08wt% of magnesium sulfate heptahydrate, and 97.09wt% of ion exchanged water), and the resulting mixture is agitated with a stirrer. The resulting gel is left in a thermo-hygrostat at 40°C × 60%RH for three hours and then cooled to room temperature. The gel strength of the gel is measured with a curdmeter (Curdmeter-MAX ME-500 manufactured by I. Techno Engineering Co., Ltd.).
- "Basis weight" is measured as follows. After preliminary drying of a sample or a test piece, the sample or the test piece is left in a test room or a test device under normal conditions (an ambient temperature of 23 ± 1°C and with a relative humidity of 50 ± 2% at the testing site) until the weight of sample or test piece reaches constant mass. Preliminary drying is to achieve the constant mass of the sample or test piece under an environment having a temperature of 100°C. For fibers having a standard moisture regain of 0.0%, preliminary drying may be omitted. The test piece having the constant mass is cut with a cutting template having the size of 100 mm × 100 mm into samples having the size of 100 mm × 100 mm. The weight of the sample is measured. The measured weight is multiplied by 100 to determine the weight per one square meter, which is defined as the basis weight.
- The "thickness" of a thick member such as the absorber 56, the absorbent element 50, or the compressed portion 51 is measured using a thickness measuring instrument of Ozaki Mfg. Co., Ltd. (Peacock, Dial Thickness Gauge, Model H (measurement range of 0 to 10 mm, circular contact point with a diameter of 10 mm, measuring force of about 1.7 N, and pressure of about 21.7 KPa) by horizontally placing the sample and the thickness measuring device.
- The "thickness" of a thin sheet such as a nonwoven fabric is automatically measured under the condition of load: 0.098 N/cm² and pressure area: 2 cm² using an automatic thickness meter (KES-G5 handy compression measurement program).
- Water absorption capacity is measured in accordance with JIS K7223-1996 "Testing method for water absorption capacity of super absorbent polymers".
- Water absorption speed is defined as "time that elapses before the end point" measured with 2g of super absorbent polymers and 50g of normal saline solution in accordance with JIS K7224-1996 "Testing method for water absorption speed of super absorbent polymers".
- "Spread state" means a state where an article is spread flat without contraction (including any contraction such as contraction by an elastic member) or slackness.
- The dimension of each component means a dimension in a spread state, not in a natural length state, unless otherwise specified.

The tests and measurements are carried out in a laboratory or apparatus under normal conditions (a temperature of 23 ± 1°C and a relative humidity of 50 ± 2% at the testing site), unless the environmental condition for the tests and measurements are otherwise specified.

### Industrial Applicability

The present invention is applicable to underpants-type disposable wearing articles such as an underpants-type disposable diaper explained in the above example.

### Reference Signs List

11 : Liquid impervious sheet
12F, 12B : Outer member
12A : Side seal portion
12B : Back outer member
12F : Front outer member
12H : Inner sheet layer
12S : Outer sheet layer
13 : Cover nonwoven fabric layer
14 : Gluteal cover portion
15 : Under-waist end elastic member
16 : Gluteal cover elastic member
17 : Waist end elastic member
200 : Inner member
201 : Inner member bonding portion
30 : Top sheet
50 : Absorbent element
51 : Compressed portion
52 : Portion with a minimum width
53 : Widening portion
54 : Portion with a maximum width
56 : absorber
56C : Central region
56S : Side region
58 : Wrapping sheet
60 : Side flap
60A : First portion
60B : Second portion
61 : Top surface-nonwoven fabric layer
62 : Underside surface-nonwoven fabric layer
631 to 633 : Gather elastic member
631 : First gather elastic member
632 : Second gather elastic member
633 : Third gather elastic member
64 : Liquid impervious film
65 : Nonwoven fabric-absent portion
66 : Nonwoven fabric
66r : Folded back portion
B : Back body part
F : Front body part
HM1 : First hot melt adhesive
HM2 : Second hot melt adhesive
L : Intermediate region
LD : Front-back direction
M : Crotch part
T : Lower torso region
U : Under-waist end portion
W : Waist end portion
WD : Width direction
WO : Waist opening
d1 : First distance
d2 : Second distance
d3 : Third distance

## Claims

1. An underpants-type disposable wearing article free of three-dimensional gather parts rising toward a top side, comprising:
an outer member, which is provided integrally from a front body part to a back body part, or which is provided separately on the front body part and the back body part;
an inner member attached to a middle part in a width direction of the outer member and provided to extend from a position on a front side of a crotch part to a position on a back side of the crotch part;
a pair of side seal portions in which both side portions of the outer member in the front body part and both side portions of the outer member in the back body part are bonded to each other, respectively;
a waist opening formed by a front edge of the front body part and a back edge of the back body part; and
leg openings provided on both lateral sides of the inner member,
wherein the inner member includes an absorber provided to be continuous from a position on the front side of the crotch part to a position on the back side of the crotch part,
the absorber includes a portion with a maximum width on the front side of the crotch part and a portion with the maximum width on the back side of the crotch part,
a pair of side flaps are provided on both side portions of the inner member to project laterally on both sides in the width direction from both side edges of the absorber and extend in a front-back direction from a front end portion of the inner member to a back end portion thereof,
each of the side flaps includes a planar gather part provided to be continuous from a lateral side of the portion with the maximum width of the absorber on the front side of the crotch part to a lateral side of the portion with the maximum width of the absorber on the back side of the crotch part,
the planar gather part includes elongated gather elastic members incorporated therein along the front-back direction such that the planar gather part is contracted in the front-back direction due to contraction of the gather elastic members and stretchable in the front-back direction due to stretching of the gather elastic members,
the side flap has a width being 0.1 to 0.5 times the maximum width of the absorber,
the gather elastic members are composed of a first gather elastic member attached on a lateral side of side edges of the portions with the maximum width of the absorber such that there is a first distance between the first gather elastic member and the side edges of the portions with the maximum width, a second gather elastic member attached on a lateral side of the first gather elastic member such that there is a second distance between the second gather elastic member and the first gather elastic member, and third gather elastic members attached repeatedly to a side edge portion of the side flap on a lateral side of the second gather elastic member such that there is a third distance for each space between two adjacent members of the second gather elastic member and the third gather elastic members,
the first gather elastic member has a first fineness and is attached to the side flap at a first stretch rate;
the second gather elastic member has a second fineness and is attached to the side flap at a second stretch rate,
each of the third gather elastic members has a third fineness and is attached to the side flap at a third stretch rate,
the second distance is 1.5 to 5 times the third distances
the first distance is 0.3 to 0.8 times the second distance, and
the first stretch rate is 0.95 to 1.05 times the second stretch rate and is 0.95 to 1.05 times the third stretch rate and at the same time, the first fineness is 1.2 to 1.5 times the second fineness and is 1.2 to 1.5 times the third fineness, the first fineness is 0.95 to 1.05 times the second fineness and is 0.95 to 1.05 times the third fineness and at the same time, the first stretch rate is 1.2 to 1.5 times the second stretch rate and is 1.2 to 1.5 times the third stretch rate, or the first fineness is 1.2 to 1.5 times the second fineness and is 1.2 to 1.5 times the third fineness and at the same time, the first stretch rate is 1.2 to 1.5 times the second stretch rate and is 1.2 to 1.5 times the third stretch rate.

2. The underpants-type disposable wearing article according to claim 1,
wherein the absorber has a portion with a minimum width in the crotch part, and widening portions, one of which is provided between the portion with the minimum width and the portion with the maximum width on the front side so as to widen forward gradually, and the other of which is provided between the portion with the minimum width and the portion with the maximum width on the back side so as to widen backward gradually; and
a distance in the width direction between a side edge of the portion with the minimum width of the absorber and the first gather elastic member is 1.1 to 1.5 times the second distance.

3. The underpants-type disposable wearing article according to claim 1 or 2,
wherein, a section, which is located between the first gather elastic member and the second gather elastic member, has a three layered structure formed of a top surface-nonwoven fabric layer, an underside surface-nonwoven fabric layer, and a liquid impervious film disposed therebetween;
the top surface-nonwoven fabric layer and the liquid impervious film are bonded to each other through a first hot melt adhesive;
the underside surface-nonwoven fabric layer and the liquid impervious film are bonded to each other through a second hot melt adhesive;
each of the top surface-nonwoven fabric layer and the underside surface-nonwoven fabric layer has a bending resistance of 0.2 to 2.2 mN.cm in the front-back direction and 0.05 to 0.5 mN.cm in the width direction according to 41.5° Cantilever Method defined by JIS L 1913: 2010;
the liquid impervious film has a bending resistance of 0.006 to 0.05 mN.cm in the front-back direction LD and 0.006 to 0.05 mN.cm in the width direction WD according to 41.5° Cantilever Method defined by JIS L 1913: 2010;
an application amount of the first hot melt adhesive is 1 to 10 g/m²; and
an application amount of the second hot melt adhesive is 1 to 10 g/m².

4. The underpants-type disposable wearing article according to claim 1 or 2
wherein the absorber has a portion with a minimum width in the crotch part, and widening portions, one of which is provided between the portion with the minimum width and the portion with the maximum width on the front side so as to widen forward gradually, and the other of which is provided between the portion with the minimum width and the portion with the maximum width on the back side so as to widen backward gradually; and
when the underpants-type disposable wearing article is placed on a horizontal plane while the back body part is located on a bottom side and only the outer member is stretched from a natural length state to a spread state along the horizontal plane for viewing the underpants-type disposable wearing article from above,
in an outer shape-line of the crotch part, an acute intersecting angle α between the front-back direction and an imaginary line connecting a position of a side edge of the absorber and a position of the first gather elastic member is 15 to 40 degrees, an acute intersecting angle γ between the front-back direction and an imaginary line connecting two positions of the third gather elastic members adjacent to each other is 50 to 90 degrees, and an acute intersecting angle β between the front-back direction and an imaginary line connecting the position of the first gather elastic member and a position of the second gather elastic member keeps a relationship: α<β<γ.
